Europäisches Patentamt

(19) European Patent Office

Office européen des brevets

(11) Publication number: **0 169 441**
**B1**

(12) **EUROPEAN PATENT SPECIFICATION**

(45) Date of publication of patent specification: **16.03.88**

(21) Application number: **85108594.4**

(22) Date of filing: **10.07.85**

(51) Int. Cl.⁴: $C\ 07\ C\ 79/10$, $C\ 07\ C\ 76/02$

(54) **Production of dinitrotoluene.**

(30) Priority: **13.07.84 US 630788**

(43) Date of publication of application:
**29.01.86 Bulletin 86/05**

(45) Publication of the grant of the patent:
**16.03.88 Bulletin 88/11**

(84) Designated Contracting States:
**CH DE IT LI SE**

(56) References cited:
**US-A-4 028 425**

(73) Proprietor: **AIR PRODUCTS AND CHEMICALS, INC.**
**Route no. 222**
**Trexlertown Pennsylvania 18087 (US)**

(72) Inventor: **Carr, Richard Van Court**
**416 S. 26th Street**
**Allentown, PA 18104 (US)**
Inventor: **Toseland, Bernard Allen**
**720 N. 26th Street**
**Allentown, PA 18104 (US)**

(74) Representative: **Dipl.-Ing. Schwabe, Dr. Dr. Sandmair, Dr. Marx**
**Stuntzstrasse 16**
**D-8000 München 80 (DE)**

Courier Press, Leamington Spa, England.

## Description

### Technical Field

This invention pertains to a process for the production of dinitrotoluene from mononitrotoluene by the reaction of the nitrotoluene with a nitrating medium comprising nitrogen dioxide and oxygen.

### Background of the Invention

Dinitrotoluene is commercially manufactured by the nitration of toluene using the mixed acid technique, i.e., a mixture of nitric acid and sulfuric acid as the nitrating medium. The product has substantial uses as an industrial chemical in the preparation of toluenediamine and toluene diisocyanate which are widely used in the manufacture of polyurethanes. Nitrogen oxides in combination with sulfuric acid and in place of nitric acid have been suggested as a co-reactant to generate nitroaromatics. By this process nitric acid is generated in situ.

Representative patents which illustrate the mixed acid technique for preparing nitroaromatics, including both mononitro and dinitroaromatics, such as mono and dinitrobenzene and mono and dinitrotoluene, are U.S. 3,256,999; 2,370,558; 2,773,911; 2,849,497; 3,185,738; 3,434,802; 4,021,498; and 4,123,466. Although there are variations within the nitration techniques described in these patents, the general method of producing the dinitrated aromatic compounds is to effect mononitration of the aromatic compound, separate the spent acid phase from the organic phase containing mononitroaromatics and then nitrate the mononitroaromatics in a dinitration zone. Because dinitration is more difficult to achieve than mononitration, higher concentrations of nitric acid and more severe nitration conditions are employed.

Although the mixed acid technique has been the primary commercial technique for the production of nitroaromatic composition, the nitration of aromatic compounds has also been effected through the use of nitric acid alone. One of the advantages of using nitric acid alone in the manufacture of nitroaromatics is that it avoids the problem of separation and reconcentration of the sulfuric acid for further use. This is a sizeable problem since the capital costs, as well as the energy costs, associated with the recovery of sulfuric acid are considerable. For example, corrosion resistant storage and transport equipment must be utilized for handling sulfuric acid. In addition, significant environmental problems are associated with the reconcentration of spent sulfuric acid.

Representative patents showing the use of nitric acid alone for producing nitroaromatics include U.S. 2,362,743, 2,739,174 and 4,028,425.

U.S. 2,362,743 at column 1, lines 5—15 and U.S. 2,739,174 at the bottom of column 1, lines 60—72 and the top of column 2, lines 1—15 refers to some of the problems associated with the mixed acid techniques. These patents generally show that when nitric acid is used alone mononitration can be achieved with a more dilute nitric acid and dinitration. For example, the '743 uses 70% nitric acid and a mole ratio of $3\frac{1}{2}$ mols nitric acid per mol toluene to achieve mononitration of toluene. Dinitration is achieved by using 98% nitric acid with a mol ratio being 3 mols nitric acid per mol of mononitrotoluene feed.

U.S. 4,028,425 differs slightly in the approach taken for the nitration of the hydrocarbon in the '743 patent. In the '425 patent nitration is effected in a reaction medium containing dinitrotoluene and 93 to 103% nitric acid. Conditions are maintained by continuously introducing a feed mixture consisting of toluene, nitrogen dioxide and oxygen in a mol ratio of at least 4 mols nitrogen dioxide per mol of oxygen per mol of toluene into the reaction zone. The reaction product then is continuously removed from the reaction vessel.

The nitration of aromatic compounds to form both mono and dinitroaromatic compounds using nitric acid alone is attractive from the point of view that it would eliminate the problems associated with the handling of sulfuric acid both in terms of capital and energy costs. However, even though the process is attractive from that standpoint, the prior art processes have been commercially unattractive primarily because of their inefficiency in terms of nitric acid consumption and oxidation by products. For example, in many dinitration reactions only about 20 mol percent of 100% nitric acid originally available is utilized. This inefficiency requires high recycle of the spent nitric acid from the nitration zones to reconcentration units. Further, as the concentation of nitric acid falls below 90% by weight, the dinitration rate drops off dramatically.

A second problem associated with possible commercial nitration of aromatic compounds with nitric acid is that the product is difficult to separate from the reaction product. The reaction product is soluble in nitric acid and therefore homogeneous. The system is unlike the mixed acid process where the reaction product is insoluble or heterogeneous and one in which the nitroaromatic compound can be separated by simple decantation. In order to extract the dinitroaromatic compound from the nitric acid process utilizing nitric acid alone, a hazardous distillation technique must be used or the nitric acid neutralized to remove product nitroaromatic compound dissolved in the nitric acid. The latter approach, of course, is not realistic in commercial operations.

### Summary of the Invention

This invention pertains to an improved process for the production of dinitrotoluene from mononitrotoluene by effecting the nitration of mononitrotoluene using concentrated nitric acid as the nitrating medium and maintaining the concentration of nitric acid in the nitration step by the introduction of nitrogen dioxide and oxygen into the nitration medium. The nitration is carried out under a temperature sufficient to effect dinitration of the mononitrotoluene, but at a temperature less than 80°C.

Several advantages can be achieved by the admission of nitrogen dioxide/oxygen mixtures to the nitration process. For example, one avoids the production of substantial amounts of the ortho-dinitrotoluene isomer and avoids the problems of the aromatic oxidation. Another is that the rate of dinitration can be maintained at a high level because of the ability to maintain a high concentration of nitric acid. A third advantage is that nitrogen dioxide, which is a coproduct of the nitric acid decomposition, can be recycled to the nitration zone. A fourth is that a runaway reaction can be controlled by venting oxygen from the reactor thereby resulting in rapid dilution of the nitric acid.

Detailed Description of the Invention

The nitratable feedstock for dinitration consists essentially of mononitrotoluene, that is, a feedstock containing from 90 to 100 percent by weight mononitrotoluene. If nitratable, but non nitroaromatic compositions are present e.g., toluene in higher concentration, the nitration proceeds, but also one experiences increased conversion of the aromatic compound to oxidation byproducts.

The mononitrotoluene suited as a feedstock can be prepared by conventional techniques such as for example, the mixed acid technique or techniques involving the nitration with nitric acid alone. If the mixed acid technique is used, the product is simply separated after mononitration and the mononitrotoluene charged to the dinitration zone. Alternately, the mononitrotoluene may be formed by effecting nitration with 75—85% nitric acid and then this product charged to a dinitration zone without separation. The nitric acid then is concentrated in situ by the addition of nitrogen oxide and oxygen. There is an additional advantage to utilizing a feedstock low in nitratable, non nitroaromatic and that is high, e.g., greater than 50°C can be used in the nitration. When the nitratable, non nitroaromatic compounds are present the undesirable meta isomer is formed in high yield, thus leading to the undesirable 2,3 3,4 3,5 and 3,6 isomers of dinitrotoluene.

Dinitration of the mononitrotoluene is carried at a temperature sufficient to effect dinitration but at a temperature of 80°C or less. Typically, the reaction is carried out at a temperature of from 40—60°C as this provides for excellent reaction rates with substantially no oxidation of the aromatic compound. Pressures used for effecting nitration range from 41 to 69 bar (600 to 1000 psig) and preferably 41 to 55 bar (600 to 800 psig). When the pressure is less than 600 psig the reaction rate is low, presumably because of insufficient reaction between the nitrogen oxide and oxygen in the reaction medium to form nitrogen dioxide. Because of the ability to decrease the oxygen supply via venting, one can reduce the hazards of dinitration in a homogeneous mixture at elevated temperatures.

To summarize, the dinitration reaction medium then comprises a nitroaromatic consisting essentially of mononitrotoluene as the feed, nitric acid, water and gaseous nitrogen oxide and oxygen. If the level of non nitro aromatic compound in the nitration zone is greater than 10%, e.g. 20% by weight, then severe oxidation of the feedstock may be encountered and substantial by-product produced. The concentration of nitric acid in the medium should range from 88 to 98% by weight, preferably 92—95%, (on an organic free basis), and this range may be generated in situ or maintained by the addition of nitrogen oxide and oxygen. At lower concentrations of nitric acid the rate of dinitration is negligible.

In order to maintain the concentration of nitric acid in the medium at this level, nitrogen dioxide and oxygen are added in a molar ratio of from 2 to 8:1, preferably 2—4:1, respectively. Optionally, nitric oxide can be injected by itself or along with nitrogen dioxide to form nitrogen dioxide in situ and subsequently react to form nitric acid. For purposes of this invention then, nitric oxide is equivalent to nitrogen dioxide, the only difference being in the amount of oxygen required. To minimize oxidation of the nitratable non nitro hydrocarbon compound, if present, from about 0.9—1 times the stoichiometric amount of oxygen required to oxidize either nitrogen dioxide or nitric oxide is used in the reaction process. Higher levels of oxygen greater than the stoichiometric quantity serve no useful purpose and can lead to oxidation by-products of the non nitroaromatic compound.

The following examples are provided to illustrate the further embodiments fo the invention and are not intended to restrict the scope thereof. All parts are parts by weights and all percentages are expressed as weight percentages.

Example 1

Into a 300 milliliter stainless steel autoclave equipped with a mechanical stirrer, cooling coils, a thermocouple, a pressure gauge and gas inlet and outlet ports there was charged 80 grams of 70 weight percent nitric acid, 140 grams of liquid dinitrogen tetroxide and 4 grams of mononitrotoluene. After charging, the autoclave was sealed and the pressure raised to 55 bar (800 psig) by the addition of oxygen. The reactants were stirred and the temperature raised to 60°C and held for 15 minutes. At that time the autoclave was vented and the contents poured onto 200 grams of ice. The cooled reaction production then was extracted twice with 25 milliliter aliquots of methylene chloride and dried over anhydrous sodium sulfate. The product then was evaporated to give 5.3 grams of a solid which when analyzed by GC contained 99.7 mol percent dinitrotoluene and 0.3 mol percent mononitrotoluene. There was no detectable trinitrotoluene formed.

This example shows that dilute nitric acid can be readily concentrated to a sufficient strength for dinitration by the addition of nitrogen dioxide and oxygen.

### Example 2

The process of Example 1 was repeated except that no oxygen was introduced to the reaction zone. After approximately 25 minutes the reaction was vented. No reaction was observed.

### Example 3

A series of reactions were carried out in accordance with the general procedure of Example 1. The basic difference is that no mononitrotoluene was added to the initial charge, the mononitrotoluene being added approximately 15 minutes after the addition of nitrogen dioxide and oxygen. The temperatures were varied for the separate runs prior to and subsequent to the introduction of mononitrotoluene. The reaction product was analyzed for percent conversion to dinitrotoluene.

Run (a) was conducted under conditions such that the initial charge and final monotoluene charge were maintained at 60°C. The conversion was 99% to dinitrotoluene.

Run (b) was run with the initial charge at 20°C and the final charge at 60°C. The conversion was 43.6%. It is believed the low conversion was caused by inadequate nitric acid generation.

Run (c) was run at 60°C for the initial charge and 10°C for the dinitration. The conversion was 97.7% thus showing good conversion to nitric acid at the 60°C level and good conversion to dinitrotoluene even at low (10°C) reaction temperature.

In the above runs, dinitration could be stopped almost immediately by terminating introduction of nitrogen oxide or by venting oxygen or both. The nitric acid concentration drops rapidly under the reaction conditions to a level that is no longer effective.

### Example 4

The dinitration of mononitrotoluene was accomplished in a continuous mode by charging 11,381 kg (25,091 pounds) per hour of mononitrotoluene and 5,233 kg (11,538 pounds) per hour of nitric acid and 1,496 kg (3,300 pounds) of water to a stirred tank suitable for dinitration. Oxygen was added at the rate of 1,152 kg (3,335 pounds) per hour and nitrogen dioxide was added at the rate of 8,695 kg (19,170 pounds) per hour to the reactor. The dinitration reactor zone was maintained at a pressure from about $4.19 \times 10^6$ to $5.58 \times 10^6$ KPa (600—800 psig) and a temperature of about 77°C (170°F). The residence time in the reactor was approximately 20 minutes. A reaction product was removed from the dinitration zone which comprised 15,119 kg (33,333 pounds) dinitrotoluene, 11,908 kg (26,254 pounds) nitric acid and 1,290 kg (2,846 pounds) of water on a per hour basis. This product was charged to a separator wherein the product was separated by decomposing the residual nitric acid.

### Claims

1. In a process for the production of dinitrotoluene from mononitrotoluene by effecting nitration of a mononitrotoluene containing feedstock using concentrated nitric acid as the nitrating medium, the improvement which comprises:

utilizing a feedstock consisting essentially of mononitrotoluene and incorporating nitrogen dioxide and oxygen into the nitration medium under conditions sufficient to generate nitric acid in situ to a concentration sufficient to effect dinitration of the mononitrotoluene; and maintaining a temperature of 80°C or less during said dinitration.

2. The process of Claim 1 wherein the feedstock to be nitrated contains from 90 to 100% by weight mononitrotoluene in the feedstock.

3. The process of Claim 2 wherein the molar concentration of nitrogen dioxide to oxygen is from 8:1 to 2:1.

4. The process of Claim 3 wherein the mixture of nitrogen dioxide and oxygen is added in an amount from 4:1 to 2:1 moles per mole of nitric acid in the nitrating medium.

5. The process of Claim 4 wherein the temperature utilized in the dinitration process is from 40 to 80°C.

### Patentansprüche

1. Verfahren zur Herstellung von Dinitrotoluol aus Mononitrotoluol durch Nitrierung eines Mononitrotoluol enthaltenden Ausgangsmaterials unter Verwendung von konzentrierter Salpetersäure als Nitriermittel, dadurch gekennzeichnet, daß man ein Ausgangsmaterial verwendet, das im wesentlichen aus Mononitrotoluol besteht und bei dem man Stickstoffdioxid und Sauerstoff in das Nitriermedium unter Bedingungen einbringt, die ausreichen, um Sal-Petersäure in situ zu einer Konzentration zu erzeugen, die ausreicht, um die Dinitrierung des Mononitrotoluols zu bewirken und bei dem man eine Temperatur von 80°C oder weniger während der Dinitrierung aufrecht erhält.

2. Verfahren nach Anspurch 1, worin das Ausgangsmaterial, das nitriert wird 90 bis 100 Gewichtsprozent Mononitrotoluol enthält.

3. Verfahren nach Anspurch 2, worin die molate Konzentration von Stickstoffdioxid zu Sauerstoff von 8:1 bis 2:1 beträgt.

4. Verfahren nach Anspruch 3, worin die Mischung aus Stickstoffdioxid und Sauerstoff in einer Menge von 4:1 bis 2:1 Mol pro Mol Salpertersäure im Nitriermedium zugegeben wird.

5. Verfahren nach Anspruch 4, worin die Temperatur, die im Dinitrierungsverfahren verwendet wird, 40 bis 80°C beträgt.

### Revendications

1. Procédé de production de dintrotoluène à partir de mononitrotoluène dans lequel on effectue la nitration d'une alimentation contenant du mononitrotoluène en utilisant de l'acide nitrique concentré comme milieu de nitration, caractérisé en ce qu'on utilise une alimentation consistant essentiellement en du mononitrotoluène et l'on incorpore du dioxyde d'azote et de l'oxygène dans le milieu de nitration dans des conditions suffisantes pour engendrer de l'acide nitrique in situ et à une concentration suffisante pour effectuer la dinitration du mononitrotoluène, tout en maintenant une température de 80°C ou moins au cours de la dinitration.

2. Procédé selon la revendication 1, caractérisé en ce que l'alimentation à nitrer contient 90 à 100% en poids de mononitrotoluène dans l'alimentation.

3. Procédé selon la revendication 2, caractérisé en ce que la concentration molaire de dioxyde d'azote par rapport à l'oxygène est de 8:1 à 2:1.

4. Procédé selon la revendication 3, caractérisé en ce que le mélange de dioxyde d'azote et d'oxygène est ajouté en une quantité de 4:1 à 2:1 moles par mole d'acide nitrique dans le mileu de nitration.

5. Procédé selon la revendication 4, caractérisé en ce que la température utilisée dans le procédé de dinitration est de 40 à 80°C.